# EUROPEAN PATENT SPECIFICATION

(11) **EP 2 160 213 B1**
(45) Date of publication and mention of the grant of the patent: **09.10.2013**
(21) Application number: 08762737.8
(22) Date of filing: 29.05.2008
(51) Int. Cl.: A61M 1/36, A61M 39/24, A61M 1/34

(54) **Kit for the extracorporeal treatment of a biological fluid of a patient, and unit provided with said kit**
Wegwerfset zur extrakorporalen Behandlung einer biologischen Flüssigkeit eines Patienten und damit ausgestattetes Gerät
Ensemble jetable pour le traitement extracorporel d'un fluide biologique d'un patient, et unité dotée dudit ensemble

(30) Priority: 29.05.2007 IT VR20070075
(43) Date of publication of application: 10.03.2010
(73) Proprietor: Haemotronic S.p.A., Mirandola (MO) (IT)
(72) Inventor: GOLDONI, Marco, I-Mirandola (MO) (IT); RAVIZZA, Alice, I-Mirandola (MO) (IT)
(74) Representative: Mola, Edoardo
(86) International application number: PCT/IB2008/001361
(87) International publication number: WO 2008/146144

(56) References cited:
- US-A- 4 012 178
- US-A- 4 141 379
- US-A- 5 423 738
- US-A- 5 992 462
- US-B1- 6 585 675

## Description

### TECHNICAL FIELD

The present invention relates to a unit for extracorporeal treatment of a biological fluid of a patient, such as, for example, for the treatment of haemodialysis.

### BACKGROUND ART

A machine for dialysis generally comprises a device for generating the pressure, for example an electronically controlled peristaltic pump, a replaceable kit, i.e., one that is both disposable and sterilizable, which can be connected in a releasable way to the pump within which the blood to be treated flows, a device for controlling the blood flow within the kit, and an electronic control unit for controlling the pump and the device for controlling the flow in a co-ordinated way.

By means of a purposely designed series of tubes, the kit defines a fluid circuit for the blood and comprises: an inlet port designed to be connected to the arterial circuit of the patient; a filter for treating the blood; and an outlet port for introducing the blood into the venous circuit of the patient.

Generally, the device for controlling the flow comprises a couple of electronically operated clips that press selectively on the wall of a tube of the kit for enabling the blood to circulate in a single direction within the fluid circuit. In particular, the control unit must be programmed for coordinating the movement of the clips with that of the pump.

A unit for dialysis according to what has been described above is effective but costly. Furthermore, a peristaltic pump which proves difficult to manage cannot be easly used in certain hospital departments, thus limiting the use of filtration technology to specialized departments, such as, for example, dialysis rooms or transfusion centres. The peristaltic pump is likewise a means that subjects both the fluid and the tube to a marked mechanical stress, and the system, to function, depends upon a source of external energy that is hence not activatable manually in the event of need.

US-A-4012178 discloses a kit according to the preamble of claim 1.

### DISCLOSURE OF INVENTION

The aim of the present invention is to provide a disposable and replaceable kit for the extracorporeal treatment of a biological fluid of a patient and a corresponding unit that is free from the drawback specified above.

The aim of the present invention is achieved via a replaceable disposable kit according to Claim 1.

### BRIEF DESCRIPTION OF THE DRAWINGS

For a better understanding of the present invention, said kit is further described also with reference to the attached figures, wherein:
- Figure 1 is a plan view of a replaceable disposable kit according to the present invention; and
- Figures 2 and 3 are longitudinal sections of a component of the kit of Figure 1.

### BEST MODE FOR CARRYING OUT THE INVENTION

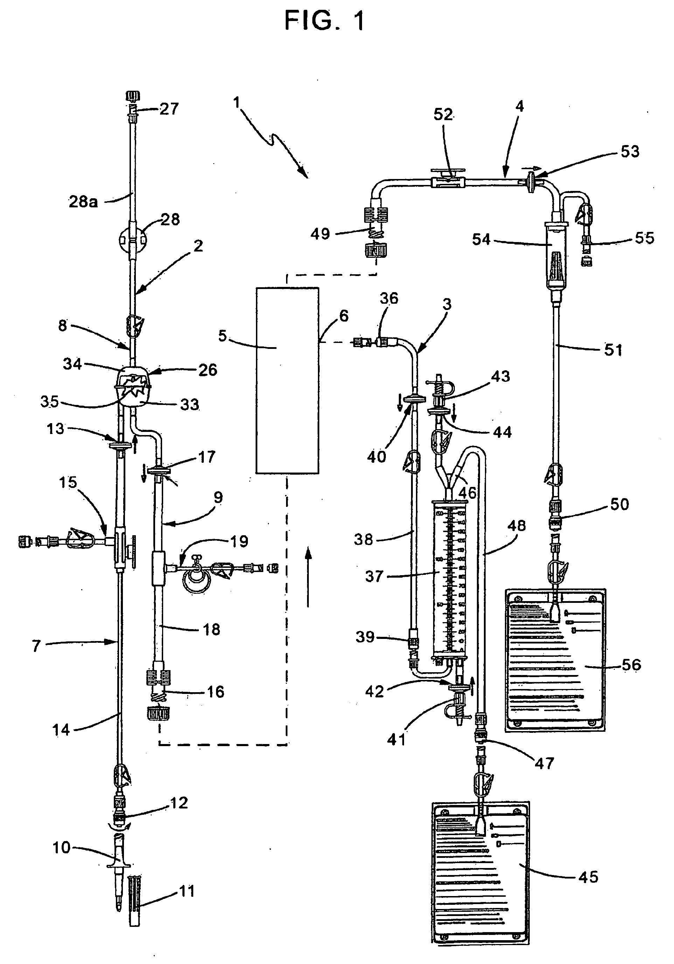

In Figure 1, designated as a whole by 1 is a kit for the extracorporeal treatment of blood or other biological fluid of a patient, comprising an actuating assembly 2 designed to be connected to an access of the patient, a display and accumulation assembly 3 for the filtered elements, for example plasmatic water, of the extracorporeal treatment, and a re-introduction assembly 4, designed to be connected to the venous system of the patient for introducing treated blood. The kit 1 is associated to a reciprocating pump (not illustrated), connected to the actuating assembly 2 and to a filter for extracorporeal treatment 5 that is traversed by the flow of fluid, for example, blood for a treatment of dialysis, directed by the actuating assembly 2 to the re-introduction assembly 4, and defines a port 6 that can be connected to the display and accumulation assembly 3.

Both the filter 5 and the pump are independent of the kit 1. Furthermore, the pump can be automatic and governed by an electronic control unit, or else manual. Preferably, the pump is a reciprocating pump and envisages a cyclic variation of constant amplitude, the value of which may be adjustable, of the volume of a variable-volume chamber. In this way, manual actuation is found to generate a known flow rate, and automatic actuation requires a simple software control. Preferably, the pump comprises a syringe.

The actuating assembly 2 comprises an intake branch 7, an activation assembly 8, and a delivery branch 9. The intake branch 7 and the delivery branch 9 are in series with respect to one another, whilst the activation assembly 8 is set between the intake branch 7 and the delivery branch 9.

In particular, the intake branch 7 comprises a perforator 10 with the corresponding cap 11 for perforating the seal of a container of sterile priming liquid (not illustrated), a 'luer' connection 12 on which the perforator 10 can be screwed, a non-return valve 13 connected upstream of the actuating assembly 8, a duct 14 for connecting the connection 12 to the non-return valve 13, and a bypass assembly 15 for enabling access to the intake branch 7 after the latter has been connected to the access to the fluid of the patient.

The delivery branch 9 comprises a connection 16 that can be connected to the filter for extracorporeal treatment 5, a non-return valve 17 connected downstream of the actuating assembly 8, a duct 18 for connecting the connection 16 to the non-return valve 17 and a bypass assembly 19 for enabling access to the delivery branch 9 after the latter has been connected to the arterial system of the patient.

In particular, the non-return valves 13, 17 are traversed in use by the flow of biological fluid and are consequently in line with the respective ducts 14, 18. They are moreover set so as to conduct the flow in a single direction, i.e., from the intake branch 7 to the delivery branch 9 (indicated by the arrows), and to prevent any reversal of the direction of the flow.

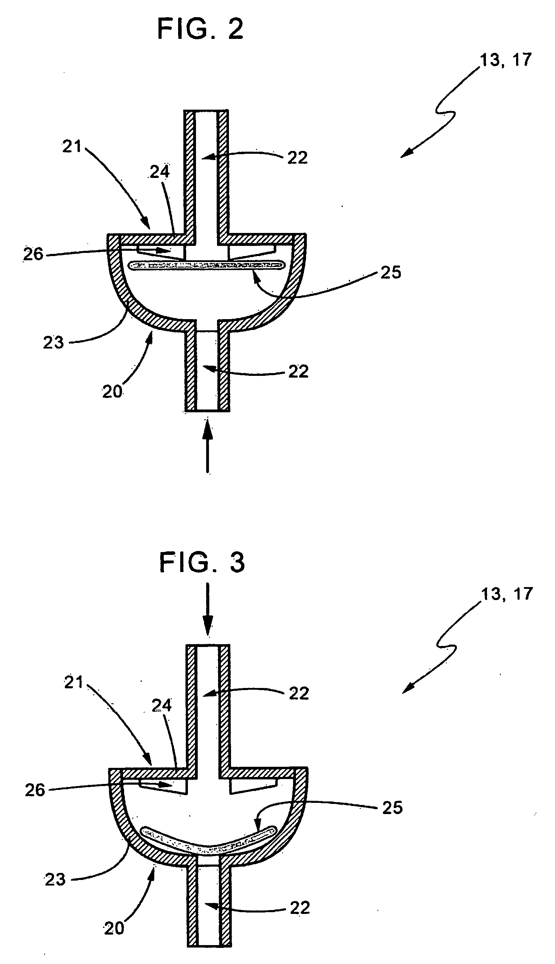

Preferably, the non-return valves 13, 17 (Figures 2 and 3) comprise an external casing having a first half-shell 20 and a second half-shell 21, connected hermetically to one another, each of which comprises a tubular engagement portion 22. The upstream half-shell 20 has a flared wall 23 coming out of the engagement portion 22, and the downstream half-shell 21 has a substantially plane wall 24 coming out of the engagement portion 22. Each non-return valve 13, 17 moreover comprises a membrane 25 made of elastomeric material mounted floating within the external casing.

In addition, the downstream half-shell 21 defines a plurality of axial projections 26, for limiting the travel of the membrane 25 in the direction of advance of the flow. According to a preferred embodiment of the present invention, each axial projection 26 has a side inclined in the direction of the flaring of the half-shell 20; for example, it has the shape of a right trapezium and is set in such a way that the inclined side faces the upstream half-shell 20. The structure thus constituted favours the passage of possible corpuscles for the purpose of guaranteeing continuity of operation of the valve. The actuating branch 8 comprises: a membrane fluid element 26 connected between the non-return valves 13, 17; a connection luer 27, designed to be connected to the pump; a duct 28a for connecting the membrane fluid element 26 to the connection 27; and a tap 28 for a pressure sensor. Preferably, the tap 28 is in line with the duct 28a.

The membrane fluid element 26 comprises a first half-shell 33 and a second half-shell 34, and a membrane 35 pinched between the half-shells 33, 34. The half-shell 33 is connected via respective ports to the intake branch 7 and to the delivery branch 9, and the half-shell 34 is connected to the duct 28a. The membrane 35 separates the duct 28a fluidically from the intake branch 7 and the delivery branch 9. The membrane 35 is made of an elastomeric material and undergoes deformation so as to transfer the pressure signals coming from the pump via the duct 28a.

The filter for extracorporeal treatment 5 is of a known type and not described further hereinafter.

The display and accumulation assembly 3 is connected to the filter 5 for displaying and accumulating the amount of filtrate separated from the blood by the filter itself.

The display and accumulation assembly 3 comprises: a 'luer' connection 36 that can be connected to the port 6 of the filter for extracorporeal treatment 5; a graduated and transparent container 37; a duct 38 for connecting the connection 36 to the container 37; a 'luer' connection 39 for connecting the duct 38 to an inlet of the container 3.7; and a non-return valve 40 that is preferably the same as the non-return valves 13, 17, set in line along the duct 38.

The graduated container moreover comprises a regulated upstream port 41 having a non-return valve 42 and a downstream port 43, which also has a non-return valve 44.

The display and accumulation assembly 3 moreover comprises a sac 45 connected to an outlet 46 of the container 37 via a 'luer' connection 47 and a duct 48. Preferably, the duct 48 is connected in a non-releasable way to the outlet 46.

The re-introduction assembly 4 is connected to the outlet of the filter for extracorporeal treatment 5 and re-introduces the treated blood into the blood system of the patient. The re-introduction assembly 4 comprises a connection 49 upstream, a 'luer' connection 50 downstream, and a duct 51 between the connections 49 and 50. In series along the duct 51, the re-introduction assembly 4 comprises: an external tap 52 for enabling injection of substances; a non-return valve 53 that is the same as the non-return valves 13, 17; and a venous drip 54 downstream of the external tap 52 and of the non-return valve 53 and having a transparent wall. The filter 54 envisages a port 55 having a 'luer' connection that can be connected, for example, to a syringe suitable for aspiration of any air that might be present within the venous drip itself.

Use of the kit 1 is described in what follows.

The kit 1 is supplied separate from the filter for extracorporeal treatment 5 and of the pump. It is extracted from a sterile pack and connected to the filter for extracorporeal treatment 5; the port 6 of the filter for extracorporeal treatment 5 is connected to the display and accumulation assembly 3.

Via the perforator 10 a sac containing physiological solution is connected in a sterile way, and an operation of priming is performed, during which the intake branch 7, the delivery branch 9, the filter 5, and the re-introduction assembly 4 are completely filled with the physiological solution, and all the air is evacuated.

The operation of priming is performed manually by gravity by the health operator without the use of dedicated equipment. In particular, the operator raises the connection 12 vertically to a height greater than that of the connection 50, and the physiological solution reaches by gravity a sac 56 provisionally connected to the connection 50.

Next, the duct 28a is filled with physiological solution, the connection 27 is connected to the delivery of a reciprocating pump, and the connection 12 is connected to the arterial circuit of the patient after the perforator 10 has been removed.

Following upon actuation of the pump, the pulsating-pressure signal is transferred along the duct 28a via the physiological solution to the membrane 35, which undergoes deformation in an alternating way. The movement thus generated causes a cyclic variation of the volume delimited between the non-return valves 13, 17 in such a way that, also thanks to the cycles of closing and opening of the latter, an alternating flow of a stop-and-go type is generated, i.e., a flow generated via a reciprocating pump, the piston of which has at least one dead-centre position, directed from the intake branch 7 to the delivery branch 9.

Said flow enables intake of the fluid and emptying of the physiological solution into the sac 56.

Following upon the operation of priming and activation of the pump, the biological fluid starts to flow within the circuit formed by the kit 1 and by the filter for extracorporeal treatment 5 whilst the physiological solution accumulates in the sac 56.

When the biological fluid is close to the sac 56, the pump is interrupted momentarily, the sac 56 full of physiological solution is disconnected from the re-introduction assembly 4, and the connection 50 is connected to the access of the patient.

The pump is again activated, and the biological fluid flows in a single direction towards the connection 50, because, when the membrane 35 undergoes deformation following upon a differential negative pressure of the duct 28a with respect to the delivery branch, the non-return valve 13 is open and the non-return valve 17 is closed. Accordingly, when the membrane 35 undergoes deformation following upon a differential overpressure of the duct 28a with respect to the delivery branch 9, the non-return valve 13 closes, whilst the non-return valve 17 opens.

The signal of pressure within the duct 28a can be monitored via the tap 28. In the case where the pump is governed by a control unit, also the pressure signal is processed by the control unit for enabling blocking of the pump in the case where anomalous pressure values are detected.

The flow generated by the deformation of the membrane 35 leads the blood to traverse the filter for extracorporeal treatment 5 where the filtrate, i.e., the waste material from the treatment, is directed towards the display and accumulation assembly 4.

In particular, the filtrate collects in the container 37 that signals to the operator via the graduated scale the amount extracted in a passive way, i.e., without any contribution of energy or intervention of a mobile mechanical element. The container 37 fills up owing to the fact that the port 43 enables release of the air.

When the maximum level is reached, regulated by a cup valve not illustrated and internal to the container 37, the operator turns the container 37 upside down in such a way that the filtrate flows into the sac 45. The port 41 enables the container 37 to empty out by causing the air to enter, and simultaneously the non-return valve 44 closes to send the entire flow to the sac 45.

At outlet from the filter for extracorporeal treatment 5, the blood traverses the re-introduction assembly 4 to go back into circulation. The non-return valve 53 ensures that the flow is maintained in the right direction also in the case of malfunctioning of the non-return valve 17.

The advantages of the kit 1 according to the present invention are described in what follows.

The non-return valves 13, 17 set along the intake branch 7 and the delivery branch 9 simplify the kit and do not require the use of the clips governed by the control unit.

Via the container 37 the operator makes a visual check on the amount of waste material produced by the filter for extracorporeal treatment 5 without the use of instruments of active indication, such as shields or balances.

The membrane 35 enables use in a sterile way of a reciprocating pump, thus considerably reducing the costs of the unit of dialysis comprising the kit 1, the pump, and the filter for extracorporeal treatment 5.

The tap 28 set in line along the duct 28a is in contact with the physiological solution and not with the biological fluid. This leads to a measurement of the pressure in total safety for the patient and the operator since the biological fluid remains isolated.

The kit 1 can function with a single pump and have just the connection 27 for connecting up to the pump. Furthermore, the operation of priming can be performed by the operator by gravity without there being present a dedicated pump.

Finally, it is clear that modifications and variations can be made to the kit described and illustrated herein, without thereby departing from the sphere of protection of the present invention.

The pump can be activated manually and thus be independent of the electrical energy. In this way, a patient or his or her biological fluid can be treated also without using the complex traditional equipment for dialysis or transfusion. The unit comprising a manual pump, the filter for extracorporeal treatment 5, and the kit 1 is readily transportable and remains sterile.

In addition, the actuating assembly 2 also without the membrane 35 and with a manual syringe connected between the non-return valves 13, 17 can be used for pumping biological fluids also for applications different from those of the dialysis treatment. The filter for extracorporeal treatment 5 can, for example, be replaced by a sieve filter for withholding thrombi. In this case, the kit is not equipped with the display and accumulation assembly 3 and can be connected also to peritoneal compartments and can treat not blood but other biological liquids of the patient.

Furthermore, the kit 1 can be supplied in combination with the filter for extracorporeal treatment 5 and separate from the pump.

In addition, the kit 1 may not be connected directly to the patient. For example, the blood of the patient can be extracted by other machinery and purposely collected in a reservoir. The kit 1 can consequently be used for treating the blood collected in the reservoir.

## Claims

1. A replaceable disposable kit for the treatment of a biological fluid of a patient, comprising an intake branch (7) designed to be connected to the haematic circuit of a patient, an activation assembly (8) designed to co-operate with a device for generating pulsating pressure for controlling a flow of the biological fluid at least in said intake branch (7), a delivery branch (9), set downstream of said intake branch (7) and said activation assembly (8), an outlet connection element (16) connected fluidically to said delivery branch (9) and designed to be connected via said activation assembly (8) to the haematic circuit of the patient, said delivery branch (9) being designed to be connected to a means for treatment (5) of said biological fluid, a first non-return valve (13) and a second non-return valve (17) set in line, respectively, on the intake branch (7) and on the delivery branch (9), said activation portion (8) being set between said first non-return valve (13) and said second non-return valve (17), said kit being **characterized by** comprising a re-introduction assembly (4) connectable to an outlet of said means of treatment (5) said assembly (4) comprising an upstream connection (49), a first 'luer' connection (50) downstream to said upstream connection (49) and a duct (51) between said upstream connection (49) and first 'luer' connections 50) wherein along said duct (51) said re-introduction assembly (4) comprises a third non-return valve (53),
said kit further comprising an evacuation assembly (3) designed to be connected to said treatment means (5) for evacuating the waste material produced by said treatment means (5), wherein said evacuation assembly (3) comprises a second 'luer' connection (36) connectable to a port (6) of said means for treatment (5), a graduated and transparent container (37) for displaying the amount of waste produced by said treatment means (5), a further duct (38) for connecting said second 'luer' connection (36) to said display device (37) and a fourth non-return valve (40) set in line along said further duct (38).

2. The kit according to claim 1, **characterized in that** said display device (37) defines a volume of accumulation and comprises a graduated scale for reading the level of filling of said volume of accumulation.

3. The kit according to any of the preceding Claims, **characterized in that** said evacuation assembly (3) comprises an accumulation element (45) connected fluidically in series to said display device (37) and a vent port (41) for emptying the display device (37) into said accumulation element (45).

4. The kit according to any of the preceding Claims, **characterized in that** it comprises said treatment device (5) that can be connected to said delivery branch (9) and to said outlet connection element (50) and **in that** said treatment device (5) can be connected to said evacuation assembly (3).

5. The kit according to any one of the preceding claims, **characterized in that** said activation assembly (8) comprises a port (27) designed to be connected to said device for generating pulsating pressure, and an elastic element (35) sensitive to a pressure signal configured for separating said port (27) fluidically from said intake branch (7) and said delivery branch (9), the flow being allowed between said first and second non-return valves (13, 17).

6. The kit according to Claim 5, **characterized in that** it comprises a further tap (28) for measuring the pressure set on said duct (28a).

7. The kit according to any one of the preceding claims, **characterized in that** at least one of said non-return valves (13, 17, 40, 53) comprises a casing (20, 21) and a membrane (25) made of elastomeric material set floating within said casing.

8. A unit for sterile treatment of a biological fluid of a patient, **characterized in that** it comprises a kit according to any one of the preceding claims and a device for generating pulsating pressure that can be connected to said activation assembly (8).

9. The unit according to Claim 8, **characterized in that** said device for generating pulsating pressure is a manual device.

10. The unit according to any of the preceding claims, **characterized in that** said third non-return valve (53) is equal to said first and second non-return valves (13, 17).

## Patentansprüche

1. Austauschbares Wegwerfset zur Behandlung eines biologischen Fluids eines Patienten, das umfasst: einen Ansaugzweig (7), der konzipiert ist, um mit dem Blutkreislauf eines Patienten verbunden zu werden, eine Aktivierungs- bzw. Betätigungsanordnung (8), die konzipiert ist, um mit einer Vorrichtung zum Erzeugen eines pulsierenden Drucks zum Steuern eines Flusses des biologischen Fluids wenigstens in dem Ansaugzweig (7) zu erzeugen, einen Abgabezweig (9), der flussabwärtig von dem Ansaugzweig (7) und der Betätigungsanordnung (8) eingerichtet ist, ein Auslassverbindungselement (16), das über ein Fluid mit dem Abgabezweig (9) verbunden ist und konzipiert ist, um über die Betätigungsanordnung (8) mit dem Blutkreislauf des Patienten verbunden zu werden, wobei der Abgabezweig (9) konzipiert ist, um mit einem Mittel zur Behandlung (5) des biologischen Fluids verbunden zu werden, wobei ein erstes Rückschlagventil (13) und ein zweites Rückschlagventil (17), die jeweils in einer Leitung auf dem Ansaugzweig (7) und dem Abgabezweig (9) eingerichtet sind, wobei der Betätigungsabschnitt (8) zwischen dem ersten Rückschlagventil (13) und dem zweiten Rückschlagventil (17) eingerichtet ist, wobei das Set **dadurch gekennzeichnet ist, dass** es eine Wiedereinleitungsanordnung (4) aufweist, die mit einem Auslass des Behandlungsmittels (5) verbindbar ist, wobei die Anordnung (4) eine flussaufwärtige Verbindung (49), eine erste 'Luer'-Verbindung (50) flussabwärtig von der flussaufwärtigen Verbindung (49) und einen Kanal (51) zwischen der flussaufwärtigen Verbindung und der ersten 'Luer'-Verbindung (50) umfasst, wobei die Wiedereinleitungsanordnung (4) entlang des Kanals (51) ein drittes Rückschlagventil (53) umfasst, wobei das Set ferner umfasst: eine Auspumpanordnung (3), die konzipiert ist, um mit dem Behandlungsmittel (3) verbunden zu werden, um das Abfallmaterial auszupumpen, das von dem Behandlungsmittel (5) erzeugt wird, wobei die Auspumpanordnung (3) eine zweite ,Luer'-Verbindung (36) umfasst, die mit einer Öffnung (6) des Behandlungsmittels (5), einem abgestuften und transparenten Behälter (37) zum Anzeigen der Menge an Abfall, die von dem Behandlungsmittel (5) erzeugt wird, einem weiteren Kanal zum Verbinden der zweiten "Luer'-Verbindung (36) mit der Anzeigevorrichtung (37) und einem vierten Rückschlagventil (40), das in der Leitung entlang des weiteren Kanal (38) eingerichtet ist, verbindbar ist.

2. Set nach Anspruch 1, **dadurch gekennzeichnet, dass** die Anzeigevorrichtung (37) ein Ansammlungsvolumen definiert und eine abgestufte Skala zum Ablesen der Füllhöhe des Ansammlungsvolumens umfasst.

3. Set nach einem der vorhergehenden Ansprüche, **dadurch gekennzeichnet, dass** die Auspumpanordnung (3) ein Ansammlungselement (45), das über ein Fluid in Reihe mit der Anzeigevorrichtung (37) angeschlossen ist, und eine Lüftungsöffnung (41), um die Anzeigevorrichtung (37) in das Ansammlungselement (45) zu entleeren, umfasst.

4. Set nach einem der vorhergehenden Ansprüche, **dadurch gekennzeichnet, dass** es die Behandlungsvorrichtung (5) umfasst, die mit dem Abgabezweig (9) und mit dem Auslassverbindungselement (50) verbindbar ist und dass die Behandlungsvorrichtung (5) mit der Auspumpanordnung (3) verbunden werden kann.

5. Set nach einem der vorhergehenden Ansprüche, **dadurch gekennzeichnet, dass** die Betätigungsanordnung (8) eine Öffnung (27), die konzipiert ist, um mit der Vorrichtung zum Erzeugen eines pulsierenden Drucks verbunden zu werden, und ein elastisches Element (35) umfasst, das für ein Drucksignal empfindlich ist und aufgebaut ist, um die Öffnung (27) fluidmäßig von dem Ansaugzweig (7) und dem Abgabezweig (9) zu trennen, wobei der Fluss zwischen den ersten und zweiten Rückschlagventilen (13, 17) zugelassen wird.

6. Kit nach Anspruch 5, **dadurch gekennzeichnet, dass** er einen weiteren Abgriff (28) zum Messen des auf dem Kanal (28a) festgelegten Drucks umfasst.

7. Kit nach einem der vorhergehenden Ansprüche, **dadurch gekennzeichnet, dass** wenigstens eines der Rückschlagventile (13, 17, 40, 53) ein Gehäuse (20, 21) und eine Membran (25) umfasst, die aus einem elastomeren Material gefertigt ist, das schwimmend in dem Gehäuse eingerichtet ist.

8. Einheit für die sterile Behandlung eines biologischen Fluids eines Patienten, **dadurch gekennzeichnet, dass** sie ein Set nach einem der vorhergehenden Ansprüche und eine Vorrichtung zur Erzeugung eines pulsierenden Drucks umfasst, die mit der Betätigungsanordnung (8) verbunden werden kann.

9. Einheit nach Anspruch 8, **dadurch gekennzeichnet, dass** die Vorrichtung zum Erzeugen eines pulsierenden Drucks eine manuelle Vorrichtung ist.

10. Einheit nach einem der vorhergehenden Ansprüche, **dadurch gekennzeichnet, dass** das dritte Rückschlagventil (53) gleich den ersten und zweiten Rückschlagventilen (13, 17) ist.

## Revendications

1. kit jetable remplaçable pour le traitement d'un fluide biologique d'un patient, ledit kit comprenant une branche d'entrée (7) conçue pour être raccordée au circuit hématique d'un patient, un ensemble d'activation (8) conçu pour coopérer avec un dispositif de génération de pression pulsée destinée à contrôler un flux du liquide biologique au moins dans ladite branche d'entrée (7), une branche de délivrance (9), placée en aval de ladite branche d'entrée (7) et dudit ensemble d'activation (8), un élément de raccordement de sortie (16) raccordé du point de vue fluidique à ladite branche de délivrance (9) et conçu pour être raccordé, par le biais dudit ensemble d'activation (8), audit circuit hématique du patient, ladite branche de délivrance (9) étant conçue pour être raccordée à un moyen de traitement (5) dudit fluide biologique, un premier clapet anti-retour (13) et un second clapet anti-retour (17) placés en ligne, respectivement, dans la branche d'entrée (7) et dans la branche de délivrance (9), ladite section d'activation (8) étant placée entre ledit premier clapet anti-retour (13) et ledit second clapet anti-retour (17), ledit kit étant **caractérisé en ce qu'**il comprend un ensemble de réintroduction (4) pouvant être raccordé à une sortie dudit moyen de traitement (5), ledit ensemble (4) comprenant un raccordement amont (49), un premier raccordement 'luer' (50) en aval dudit raccordement amont (49) et un conduit (51) entre lesdits raccordement amont (49) et premier raccordement 'luer' (50), ledit ensemble de réintroduction (4) comprenant le long dudit conduit (51) un troisième clapet anti-retour (53), ledit kit comprenant en outre un ensemble d'évacuation (3) conçu pour être raccordé audit moyen de traitement (5) afin d'évacuer la matière résiduaire produite par ledit moyen de traitement (5), ledit ensemble d'évacuation (3) comprenant un second raccordement 'luer' (36) pouvant être raccordé à une entrée (6) dudit moyen de traitement (5), un récipient transparent et gradué (37) destiné à afficher la quantité de matière résiduaire produite par ledit moyen de traitement (5), un autre conduit (38) destiné à raccorder ledit second raccordement 'luer' (36) audit dispositif d'affichage (37) et un quatrième clapet anti-retour (40) placé en ligne le long dudit autre conduit (38).

2. kit selon la revendication 1, **caractérisé en ce que** le dispositif d'affichage (37) définit un volume d'accumulation et comprend une échelle graduée pour lire le niveau de remplissage dudit volume d'accumulation.

3. kit selon l'une quelconque des revendications précédentes, **caractérisé en ce que** ledit ensemble d'évacuation (3) comprend un élément d'accumulation (45) raccordé du point de vue fluidique en série audit dispositif d'affichage (37) et une entrée de purge (41) destinée à vider le dispositif d'affichage (37) dans ledit élément d'accumulation (45).

4. kit selon l'une quelconque des revendications précédentes, **caractérisé en ce qu'**il comprend ledit dispositif de traitement (5) qui peut être raccordé à ladite branche de délivrance (9) et audit élément de raccordement de sortie (50) et **en ce que** ledit dispositif de traitement (5) peut être raccordé audit ensemble d'évacuation (3).

5. kit selon l'une quelconque des revendications précédentes, **caractérisé en ce que** ledit ensemble d'activation (8) comprend une entrée (27) conçue pour être raccordée audit dispositif de génération de pression pulsée et un élément élastique (35) sensible à un signal de pression et configuré pour séparer ladite entrée (27) du point de vue fluidique de ladite branche d'entrée (7) et de ladite branche de délivrance (9), le flux étant autorisé entre lesdits premier et second clapets anti-retour (13, 17).

6. kit selon la revendication 5, **caractérisé en ce qu'**il comprend une prise supplémentaire (28) destinée à mesurer la pression appliquée sur ledit conduit (28a).

7. kit selon l'une quelconque des revendications précédentes, **caractérisé en ce que** au moins un desdits clapets anti-retour (13, 17, 40, 53) comprend un boîtier (20, 21) et une membrane (25) fabriquée à partir d'une matière élastomère et placée de façon flottante à l'intérieur dudit boîtier.

8. Unité de traitement stérile d'un fluide biologique d'un patient, **caractérisée en ce qu'**elle comprend un kit selon l'une quelconque des revendications précédentes et un dispositif de génération de pression pulsée qui peut être raccordée audit ensemble d'activation (8).

9. Unité selon la revendication 8, **caractérisée en ce que** ledit dispositif de génération de pression pulsée est un dispositif manuel.

10. Unité selon l'une quelconque des revendications précédentes, **caractérisée en ce que** ledit troisième clapet anti-retour (53) est identique auxdits premier et second clapets anti-retour (13, 17).
